# EUROPEAN PATENT SPECIFICATION

(11) **EP 1 694 396 B1**
(45) Date of publication and mention of the grant of the patent: **03.12.2008**
(21) Application number: 04775392.6
(22) Date of filing: 08.09.2004
(51) Int. Cl.: A61M 25/00

(54) **Catheter with pump**
Katheter mit Pumpe
Catheter avec pompe

(30) Priority: 17.12.2003 US 529763 P; 17.12.2003 SE 0303387
(43) Date of publication of application: 30.08.2006
(73) Proprietor: Skansén, Jan, 134 06 Ingarö (SE); Ulfendahl, Hans, 756 52 Uppsala (SE)
(72) Inventor: Skansén, Jan, 134 06 Ingarö (SE); Ulfendahl, Hans, 756 52 Uppsala (SE)
(74) Representative: Holmberg, Nils Anders Patrik
(86) International application number: PCT/SE2004/001287
(87) International publication number: WO 2005/058404

(56) References cited:
- US-A- 4 698 058
- US-A- 4 705 501
- US-A- 4 826 810
- US-A- 5 156 596
- US-A- 5 360 397
- US-A- 2003 216 685
- US-B1- 6 254 586

## Description

### Field of the invention

The present invention relates to a catheter device and an infusion system according to the preambles of the independent claims.

### Background of the invention

The invention relates to a catheter device adapted for administration of liquid substances from an externally carried pump device into a patient's blood or lymphatic vessels, visceral cavities or interstitial spaces.

A number of systems and methods to administer one or more active substances to humans and other mammalians are known. The active substance is administrated to a specific site with a relative high concentration in order to affect the site or to be further allocated by the circulatory system.

One problem occurring with the known systems is that, if the administrated liquid contains for example hormones or other substances, tissue growth is promoted at the target site as well as in the catheter system causing a hindrance for the liquid to be distributed. Where such a catheter system is intended for long-term use, as in the case of insulin administration, it is necessary to flush the catheter end and target site to reduce the active substance promotion of tissue growth. The flushing liquid is usually physiological saline, Ringer solution or similar fluids. This flushing procedure is both bothersome for the patient and expensive for the health care.

Another problem with the catheters used today is the fact that some biologically active substances in water solutions have a tendency to adhere to the catheter walls and then form aggregates and clots resulting in a stop flow in the catheter. Peptides are known to adhere to plastic surfaces. In this case the catheter often must be exchanged.

A third problem, in particular for subcutaneous catheter systems that penetrates the skin, is the risk of infections. Even if the risk not is substantial it becomes high in the case of many years of treatment with repeated skin penetrations. This is a particularly referred risk aspect, which has limited the utilization of external pumps for subcutaneous insulin administration.

Pulsative flows have been discussed in US-4,826,810, which specifically relates to insulin as active substance and carbohydrate as flushing liquid. In this patent a pump section is arranged that includes a pump mechanism and an insulin reservoir. An infusion catheter extends from the pump mechanism and is inserted into a patient to deliver insulin intravascularly, intraperitoneally, or subcutaneously. The system may be maintained by administering time-varying quantities of insulin by pulses of insulin, i.e. injections or infusions which start and stop within a short period of time (in the order of seconds). However, there is no disclosure in US-4,826,810 regarding any special features of the catheter.

In US-6,254,586 is disclosed a method and a kit for supplying a fluid to a subcutaneous placement site.

US 2003/0216685 A1 discloses devices and methods for delivering fluid components of n embolic mass into a body cavity. A connector, which includes a receiving element, a first port, and a second port, is provided for securing an outer tubular element and an inner tubular element such that the outer tubular element coaxially surrounds the inner tubular element. First and second fluid components of an embolic composition are delivered into the first and second ports of the connector and through lumens of the outer and inner tubular elements for occlusion of the body cavity.

Thus, one object of the present invention is to achieve a catheter device that is less bothersome for the patient to use and that requires fewer follow-up visits at the hospital and is therefore less expensive.

One further object of the invention is to achieve a catheter device where the active substance has less tendency to adhere to the catheter walls and to form aggregates and clots resulting in a stop flow in the catheter.

Still one object of the present invention is to achieve a catheter device that does not have to be exchanged as often as the prior art catheter system and thereby decreasing the risk of infection.

### Summary of the invention

The above-mentioned objects are achieved by the present invention according to the independent claim.

Preferred embodiments are set forth in the dependent claims.

The key elements of the invention are that the active substance is administrated by using a catheter device comprising an outer catheter and an inner catheter. The inner catheter is replaceable without interrupting the position of the outer catheter, the inner catheter's fixation to the outer catheter ensures that the inner catheter distal opening is positioned proximal the distal opening of the outer catheter.
Both catheters are each connected to a tubing from an external pump using a specific connection and, in the case of using a transcutaneous port, the plug to the port is connected to the outer catheter. When disconnecting the tubing and catheters sterile Teflon^{®} plugs are preferably inserted to the open ends of tubing and catheters.

According to a preferred embodiment, all surfaces in contact with the active substance in the catheter device are made of or covered by tetrafluoro polyethylene (Teflon^{®}) to prevent that aggregates and clots are formed resulting in that the flow in the catheter is restricted or stopped.

According to another preferred embodiment the catheter device enters e.g. the abdominal cavity through a transcutaneous port which is considered to strongly reduce the infection risk.
The use of a transcutaneus port is used e.g. when performing peritoneal dialysis and for catheterisation of the choledoctus branches. The port is usually made out of a biocompatible material like titanium, which outer sides promotes tissue ingrowths but keeps a hollow area where the catheter may be located. The port has a plug so that patients can swim or shower without any fear of infectious material entering the abdominal cavity.

The active liquid substance and the flushing liquid are transported from the external pump mechanism using two separate pieces of tubing to the interface to the catheter device according to the invention.

At the connection between the catheter sets, there is a female connector at the tubing coming from the pump mechanism and a male connector from the catheter device.

The inner catheter, in which one or more lumens are used to administer active substances, enters the outer catheter a few centimetres prior to the transcutaneous entrance via a catheter connector, preferably a Y connector, which is equipped with e.g. a screw tightening sealing. This makes it possible to replace and exchange the inner catheter in case of increasing flow resistance due to tissue growth at the catheter tip and to peptide aggregates adhered to the inner surface of the catheter, while having the outer catheter in place.

According to a preferred embodiment this exchange is achieved by having a male threaded connector fixated on the inner catheter, while on the catheter connector a female threaded connection is arranged having a self sealing mechanism when threaded together. The female connecting part on the outer catheter is permanently fixated so that the distance between inner catheter opening and the outer catheter opening is known when they are connected to each other. The outer catheter, which is providing the flushing liquid, protects the inner catheter at the outflow opening while when the flushing liquid cleans the inner catheter's outflow area.

Thus, when the inner catheter is fixated to the outer catheter the distance between the two catheters' outflow openings are known. Since the catheter dimensions and the distance are known, the minimal volume required to flush the active substance from the inner catheter outflow opening and into target area can be calculated. The flushing volume must, however, be chosen slightly larger because of the laminar flow. This information is a requirement in order to program a flushing sequence after each active substance pulse volume and to preclude that each pulsed volume of an active substance in a pulsating sequence is effectively washed out. Therefore, the dual-catheter using a pair design creates a prerequisite for effective administration of pulsating sequences for liquids.

In case of utilising a permanent transcutanous port, the port's sealing system is used over the outer catheter to secure the fixation and position of the catheter and to protect the entrance of infection agents.

Above construction allows the exchange of a clogged inner catheter causing an infusion stop without having to exchange the complete catheter system. It also provides the possibility that the exchange of the inner catheter may be performed in a rather simple equipped sterile environment.

By improving the easiness of exchanging the inner catheter the therapy quality of pump administration of e.g. insulin is enhanced and thereby a better treatment for a continuously growing patient group is provided.

### Short description of the appended drawings

Figure 1 shows a schematic illustration of an infusion system including a catheter device according to a first embodiment of the present invention.
Figure 2 shows a schematic illustration of an infusion system including a catheter device according to a second embodiment of the present invention.
Figure 3 shows a schematic illustration of a part of the Y-connection according to the present invention.
Figure 4 shows a schematic illustration of a distal part of the Y-connection according to the present invention.

### Detailed description of preferred embodiments of the invention

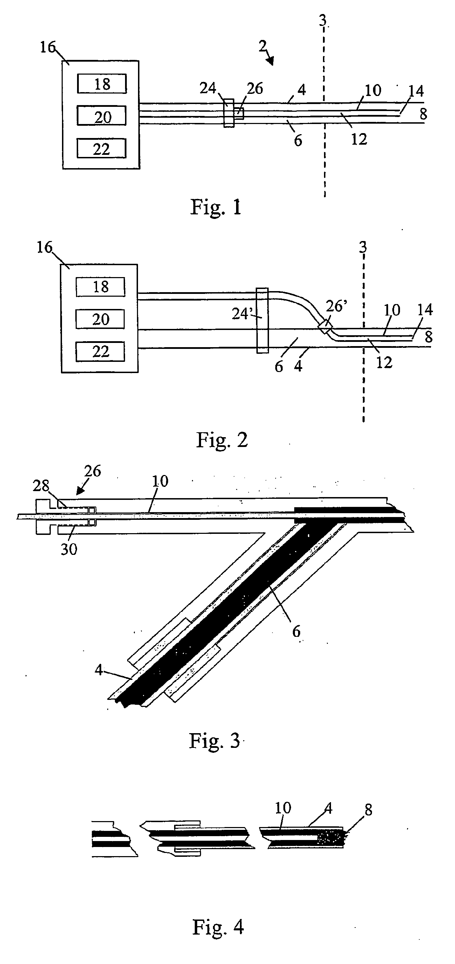

Figure 1 shows a schematic illustration of an infusion system including a catheter device according to the present invention.
In figure 1 is shown a catheter device 2 for transcutaneous or subcutaneous administration, through the skin 3, of substances to a patient, comprising an outer catheter 4 provided with one outer catheter lumen 6 with a distal outer catheter outflow opening 8 and an inner catheter 10 provided with at least one inner catheter lumen 12 with at least one distal inner catheter outflow opening 14. The inner catheter 10 is adapted to be detachably arranged in said outer catheter lumen 6. When the catheter device is adapted to be used for administration of substances to a patient, the inner catheter outflow opening 14 is located proximally said outer catheter outflow opening 8. In use the catheter device preferably has its distal end in a free abdominal cavity.

The dimensions of the catheter device is related to the specific use, e.g. where the substance is to be administrated. The overall length of the catheter device may be in the range of 5-30 cm. The diameter of the outer catheter may be 4 mm or less and the outer diameter of the inner catheter is naturally related to the inner diameter of the outer catheter and may be in the range 1-2 mm. However, other dimensions may also naturally be used.

In figure 1 is also seen an external pump device 16 including a pumping means 18, a reservoir means 20 and a control means 22. The catheter device and the external pump device together constitutes an infusion system. The reservoir means has one or more reservoirs for substances and flushing liquids.
The control means is adapted to control the pumping means such that a preset sequence of liquid pulses to be dispensed by said inner and outer catheters is obtained. The pumping means includes at least two pumps driven by motors connected to an energy source (not shown) as an example a battery, and controlled by the control means.
Preferably the pumping means may include micro pumps (not shown) driven by linear motors that gets its energy from the energy source and connected to the reservoirs for the substances.
The control means 22 is defined to secure that an active substance, for example a hormone or a cytostatikum, in the pump is distributed in programmable dosages in the micro litre range to the outflow opening 14 of the inner catheter 10 during short periods of time, for example in the range of seconds, and that these periods of primary dosages are distinguished from secondary periods which can last between 1 - 30 minutes. One distinguished character of the pumping means is that it is designed to operate with selected periods of primary dosages of 1 to 30 minutes.

The pumping means is also programmed to generate a liquid pulses of flushing liquid after each liquid pulse of an active substance where each liquid pulse dosage is related to the volume between the openings of the inner and outer catheters. The dosage of active substance released from the inner catheter opening is thereby flushed out from the space between the inner and outer opening by the flushing liquid pulse applied via the outer catheter lumen.

It is also possible to use catheter device to retrieve fluid from inside the body in order to analyze the content of fluid and to use the inner or outer catheter or enter another fluid, if so needed.

The inner catheter is preferably coaxially arranged with regard to the outer catheter.

At least one of the substances is active, such as a hormone, e.g. insulin, a peptide, an anti-thrombolytic agent or any other pharmaceutical preparation for therapeutic or diagnostic use and the active substance is preferably administered by the inner catheter.

The substance is administered as a pulsed flow sequence of substance comprising a predetermined number of liquid pulses, wherein each liquid pulse is a predetermined volume of the substance. The predetermined volume of a liquid pulse depends of many different parameters, e.g. the used substance, the concentration of the substance, where the substance is administrated.
As an exemplary embodiment, where insulin is used as an active substance, each liquid pulse has a volume in the order of 1 microliter, e.g. 0,5-1,5 microliters. The total volume for all pulses in the whole pulsed flow sequence of substance is in the interval 4-8 microliters.

The volume of a liquid pulse of the active substance is approximately the same as the volume defined in said outer catheter lumen between the inner catheter outflow opening and the outer catheter outflow opening. A liquid pulse of the active substance through the inner catheter lumen is followed in time sequence by a liquid pulse of a flushing liquid applied through the outer catheter lumen, in order to make the active substance reach the target area of administration.

The catheter device is provided with a first connector means 24 for connection to the external pump device 16 and with a second connector means 26 making it possible to detach the inner catheter and replace it if necessary.

According to a preferred embodiment the second connector means 26 is partly integrated in a Y-connection, this is further illustrated in figures 3 and 4.

The second connector means 26 includes a first fastening means at the proximal end of said inner catheter adapted to co-operate with a second fastening means 30 integrated with an opening in the outer catheter wall such that when said first and second fastening means are attached to each other the catheter device is in a substance administration state. This opening may e.g. be realised as a Y-connection as shown in figures 3 and 4.
During a replacement procedure the first fastening means 28 is detached from the second fastening means 30 and the inner catheter is withdrawn out through the opening in the outer catheter wall and a new inner catheter may be inserted through said opening. The positions of the first and second fastening means ensure that a predetermined volume is obtained in the distal end of the outer catheter.

As mentioned above the outer and inner catheters comprise at their respective proximal ends first connector means 24 arranged so that the catheter device may be disconnected from the external pump device.

The outer catheter is adapted to administer a flushing liquid. A minimal volume of flushing liquid is calculated and used to allow each pulse of the active substance in pulsed sequence to enter a target area of administration.

According to an alternative embodiment the inner catheter comprises two lumen where each lumen in the inner catheter is adapted to administer an active substance.

According to a preferred embodiment all surfaces in contact with the active substance in the catheter device are made of or covered by tetrafluoro polyethylene (Teflon^{®}) in order to avoid the aggregates or clots are formed at the catheter surfaces.

Figure 3 shows a schematic illustration of a proximal part of the Y-connection and figure 4 shows a schematic illustration of a distal part of the Y-connection according to the present invention.

In the figure 3 the inner catheter 10 enters the outer catheter 4 through a Y-connector positioned prior the skin penetration and is connected by the second connector means 26 that includes the first fastening means 28 integrated with proximal end of the inner catheter and realised e.g. as outer threads and the second fastening means 30 at the Y-connection realised e.g. as inner threads. The second connector means also ensures that the first catheter lumen is aligned proximally and distally the second connector means. The active substance to be applied via the inner catheter lumen may be identified in the figure as well as the flushing liquid (shown as black) applied via the outer catheter lumen.

In figure 4 it is shown the connection of the outer catheter to the distal part of the Y-connector and the distal part of the inner catheter that is inserted and attached to the Y-connector as shown in figure 3.

To place the catheter device 2, for example in a subcutaneous position, an opening in the skin 3 is made using an introducer, or a cannula, in combination with an introducer. The catheter device 2 is then entered into the introducer, which is penetrating skin, and when the catheter end has reached the target area, the introducer is split and removed. For abdominal cavity positioning a permanent transcutaneous port may be utilised whereby the catheter device is entered through the opening of the port where after the opening is closed using a port sealing.

Thereafter the inner catheter 10 may be entered into the outer catheter 4 positioned outside the port, and entered so far that first fastening means 28 fixated on the inner catheter can be screwed on to the outer catheter. As stated above the inner catheter has a fixated fastening means located at its length so that when connected to the Y-connector body the outflow openings 8, 14 become separated from each other. As the two catheters are one pair, the inner catheter's outflow opening 14 is automatically positioned at a defined distance short of the outer catheter's outflow opening 8. Then the catheter device may be connected to the external pump device 16.

The connection between the pump means 16 and catheter device 2 may be disconnected at exchange of catheter device or at any time the patient so prefer. Disconnection is performed by release of the first connector means 24 that comprises a pair of connector parts (not shown).
The two connector parts may be connected so that the catheter lumens for the inner and outer catheters are aligned and protected by sealing rings.

Small dosages of an active substance, in the range of micro litre, may under short primary time periods be administrated through the inner catheter 10, using a timing interval of 1 - 30 minutes between each primary time period. At, for example hormone treatment, the dosage selection is made to correspond to the amount of hormone the body's muscle cells and liver cells can absorb during the time period when these cells can absorb hormone from their environment to minimize excessive exposure of hormone to the body.

The catheter according to the present invention may be used for other active substances to other body cavities or organs. For example the catheters may be placed in Vena Porta or any of its branches.

The present invention is not limited to administration of hormones but may be utilized in administration of other pharmacological substances or other biological active substances, for example cytostatics or growth preventing drugs in cancer therapy.

In such cases the catheter device may be inserted so that the outflow openings enter a target area for treatment, for example up-streams an intestine that is target to be treated with cytostatics whereas this substance shall be administered within selected time periods, adding a solution and flushed as described above. The advantage is that the catheter device may be positioned for a longer time of treatment without the risk of over exposure of active highly concentrated substances at the area of the catheter's outflow tract.

In other cases the catheter device may be used in a vein or artery, whereby the active substance is a drug to prevent blood clotting at or around the catheter's outflow opening, whereas the other catheter can be used to administer a nutrient solution with or without pharmaceuticals.
It should be clear that the expression flushing liquid also may include a solution having an own functionality, for example circulatory vein/artery enlarging pharmaceuticals. Due to the flushing liquid it is possible to let each pulsed volume of active substance to be administered and synchronized with the cell cycle receptivity at the target area. The flushing liquid at each pulsed volume of active substance also prevents quick fibrosis growth built-up in and around the outer catheters outflow tract.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Infusion system comprising a catheter device (2) for transcutaneous or subcutaneous administration of liquid substances to a patient, comprising an outer catheter (4) provided with one outer catheter lumen (6) with a distal outer catheter outflow opening (8) and an inner catheter (10) provided with at least one inner catheter lumen (12) with at least one distal inner catheter outflow opening (14), said inner catheter is adapted to be detachably arranged in said outer catheter lumen, said inner catheter outflow opening is located proximally said outer catheter outflow opening when the catheter device is adapted to be used for administration of liquid substances to a patient **characterized in that** the infusion system further comprises an external pump device (16) including a pumping means (18), reservoir means (20) and a control means (22), and that said control means is adapted to control the pumping means such that said substance is administered as a pulsed flow sequence of liquid substance comprising a predetermined number of liquid pulses, wherein each liquid pulse is a predetermined volume of the substance, and that a liquid pulse of the liquid substance through the inner catheter lumen is followed in time sequence by a liquid pulse of a flushing liquid applied through the outer catheter lumen, in order to make the liquid substance reach an target area of administration.

2. Infusion system according to claim 1, **characterized in that** said inner catheter is coaxially arranged with regard to said outer catheter.

3. Infusion system according to claim 1, **characterized in that** at least one substance, adapted to be administered, is active, such as a hormone, e.g. insulin, a peptide, an anti-thrombolytic agent or any other pharmaceutical preparation for therapeutic or diagnostic use.

4. Infusion system according to claim 3, **characterized in that** said active substance is administered by said inner catheter.

5. Infusion system according to claim 1, **characterized in that** outer and inner catheters comprise at their respective proximal ends first connection means (24) for connection to said external pump device having one or more reservoirs for substances and flushing liquids.

6. Infusion system according to claim 1, **characterized in that** said catheter device is provided with a second connector means (26) making it possible to detach said inner catheter and replace it.

7. Infusion system according to claim 6, **characterized in that** said second connector means is partly integrated in a Y-connection.

8. Infusion system according to claim 7, **characterized in that** said second connector means includes a first fastening means (28) at the proximal end of said inner catheter adapted to co-operate with a second fastening means (30) integrated with an opening in the outer catheter wall such that when said first and second fastening means are attached to each other the catheter is in a substance administration state.

9. Infusion system according to claim 1, **characterized in that** the inner catheter comprises two lumen.

10. Infusion system according to claim 1, **characterized in that** all surfaces in contact with active substances in the catheter device are made of or covered by tetrafluoro polyethylene (Teflon^{®}).

11. Infusion system according to claim 1, **characterized in that** the volume of a liquid pulse of the substance is approximately the same as the volume defined in said outer catheter lumen between the inner catheter outflow opening and the outer catheter outflow opening.

## Patentansprüche

1. Infusionssystem, umfassend ein Kathetergerät (2) zur transkutanen oder subkutanen Verabreichung von flüssigen Substanzen an einen Patienten, umfassend einen Außenkatheter (4), der mit einem Außenkatheterlumen (6) mit einer distalen Außenkatheterausflussöffnung (8) versehen ist, und einen Innenkatheter (10), der mit zumindest einem Innenkatheterlumen (12) mit zumindest einer distalen Innenkatheterausflussöffnung (14) versehen ist, wobei der Innenkatheter dazu geeignet ist, abnehmbar in dem Außenkatheterlumen angeordnet zu sein, wobei sich die Innenkatheterausflussöffnung proximal zu der Außenkatheterausflussöffnung befindet, wenn das Kathetergerät dazu geeignet ist, zur Verabreichung von flüssigen Substanzen an einen Patienten benutzt zu werden, **dadurch gekennzeichnet, dass** das Infusionssystem ferner ein externes Pumpengerät (16) mit einem Pumpmittel (18), Behältermittel (20) und einem Steuermittel (22) umfasst, und dass das Steuermittel dazu geeignet ist, das Pumpmittel derart zu steuern, dass die Substanz als impulsartige Flusssequenz von flüssiger Substanz verabreicht wird, die eine vorgegebene Anzahl von Flüssigkeitsimpulsen umfasst, wobei jeder Flüssigkeitsimpuls ein vorgegebenes Volumen der Substanz beträgt, und dass einem Flüssigkeitsimpuls der flüssigen Substanz durch das Innenkatheterlumen in Zeitfolge ein Flüssigkeitsimpuls einer Spülflüssigkeit folgt, der durch das Außenkatheterlumen erfolgt, um die flüssige Substanz einen Zielverabreichungsbereich erreichen zu lassen.

2. Infusionssystem nach Anspruch 1, **dadurch gekennzeichnet, dass** der Innenkatheter bezüglich des Außenkatheters koaxial angeordnet ist.

3. Infusionssystem nach Anspruch 1, **dadurch gekennzeichnet, dass** zumindest eine Substanz, die zur Verabreichung geeignet ist, aktiv ist, wie etwa ein Hormon, beispielsweise Insulin, ein Peptid, ein Antithrombotikum oder jegliches andere pharmazeutische Präparat zum therapeutischen oder diagnostischen Gebrauch.

4. Infusionssystem nach Anspruch 3, **dadurch gekennzeichnet, dass** die aktive Substanz durch den Innenkatheter verabreicht ist.

5. Infusionssystem nach Anspruch 1, **dadurch gekennzeichnet, dass** Außen- und Innenkatheter an ihren jeweiligen proximalen Enden erste Verbindungsmittel (24) zum Verbinden mit dem externen Pumpengerät mit einem oder mehreren Behältern für Substanzen und Spülflüssigkeiten umfassen.

6. Infusionssystem nach Anspruch 1, **dadurch gekennzeichnet, dass** das Kathetergerät mit einem zweiten Verbindermittel (26) versehen ist, das das Abnehmen und Ersetzen des Innenkatheters ermöglicht.

7. Infusionssystem nach Anspruch 6, **dadurch gekennzeichnet, dass** das zweite Verbindermittel teilweise in einer Y-Verbindung eingegliedert ist.

8. Infusionssystem nach Anspruch 7, **dadurch gekennzeichnet, dass** das zweite Verbindermittel ein erstes Befestigungsmittel (28) am proximalen Ende des Innenkatheters enthält, das derart zum Zusammenwirken mit einem zweiten Befestigungsmittel (30), das mit einer Öffnung in der Außenkatheterwand eingegliedert ist, geeignet ist, dass sich der Katheter, wenn das erste und zweite Befestigungsmittel aneinander angebracht sind, in einem Substanzverabreichungszustand befindet.

9. Infusionssystem nach Anspruch 1, **dadurch gekennzeichnet, dass** der Innenkatheter zwei Lumen umfasst.

10. Infusionssystem nach Anspruch 1, **dadurch gekennzeichnet, dass** alle Oberflächen, die sich mit aktiven Substanzen in dem Kathetergerät in Kontakt befinden, aus Polytetrafluorethylen (Teflon®) hergestellt oder damit beschichtet sind.

11. Infusionssystem nach Anspruch 1, **dadurch gekennzeichnet, dass** das Volumen eines Flüssigkeitsimpulses der Substanz ungefähr dasselbe wie das Volumen ist, das in dem Außenkatheterlumen zwischen der Innenkatheterausflussöffnung und der Außenkatheterausflussöffnung definiert ist.

## Revendications

1. Système de perfusion comprenant un dispositif de cathéter (2) pour l'administration transcutanée ou sous-cutanée de substances liquides à un patient, comprenant un cathéter externe (4) pourvu d'une lumière (6) de cathéter externe avec au moins une ouverture distale (8) d'écroulement du cathéter externe et un cathéter interne (10) pourvu d'au moins une lumière (12) de cathéter interne avec au moins une ouverture distale (14) d'écoulement du cathéter interne, le dit cathéter interne est apte à être placé de manière détachable dans la dite lumière du cathéter externe, la dite ouverture d'écoulement du cathéter interne est située en relation proximale avec la dite ouverture d'écoulement du cathéter externe quand le dispositif de cathéter est adapté à être utilisé pour administrer des substances liquides à un patient, **caractérisé en ce que** le système de perfusion comprend de plus un dispositif externe de pompe (16) comprenant un moyen de pompage (18), un moyen de réservoir (20) et un moyen de contrôle (22) et **en ce que** le dit moyen de contrôle est adapté à contrôleur le moyen de pompage de façon à ce que la dite substance soit administrée sous forme d'une séquence de flux pulsatile de la substance liquide comprenant un nombre prédéterminé d'impulsions du liquide, où chaque impulsion du liquidé est un volume prédéterminé de la substance, et **en ce qu'**une impulsion de la substance liquide à travers la lumière du cathéter interne est suivie en séquence temporelle par une impulsion d'un liquide de chasse appliqué à travers la lumière du cathéter externe, affin de faire atteindre à la substance liquide une zone cible d'administration.

2. Système de perfusion selon la revendication 1,
**caractérisé en ce que** le dit cathéter interne est disposé de manière coaxiale par rapport au dit cathéter externe.

3. Système de perfusion selon la revendication 1,
**caractérisé en ce qu'**au moins une substance, adaptée à être administrée, est active, commue une hormone, par exemple l'insuline, un peptide, un agent anti-thrombolytique ou toute autre préparation pharmaceutique pour une utilisation thérapeutique ou diagnostique.

4. Système de perfusion selon la revendication 3,
**caractérisé en ce que** la dite substance active est administrée par le dit cathéter interne.

5. Système de perfusion selon la revendication 1,
**caractérisé en ce que** les cathéters interne et externe comprennent à leurs extrémités proximales respectives un premier moyen de connexion (24) pour la connexion au dit dispositif externe de pompe ayant un ou plusieurs réservoirs pour les substances ou les liquides de chasse.

6. Système de perfusion selon la revendication 1,
**caractérisé en ce que** le dit dispositif de cathéter est pourvu d'un deuxième moyen de connexion (26) qui rend possible de détacher le dit cathéter interne et de le remplacer.

7. Système de perfusion selon la revendication 6,
**caractérisé en ce que** le dit deuxième moyen de connexion est partiellement intégré dans une connexion en étoile.

8. Système de perfusion selon la revendication 7,
**caractérisé en ce que** le dit deuxième moyen de connexion comprend un premier moyen de fixation (28) à l'extrémité proximale du dit cathéter interne adaptée à coopérer avec un deuxième moyen de fixation (30) intégré dans une ouverture dans la paroi du cathéter externe de façon à ce que quand les dits premier et deuxième moyen de fixation sont attachés l'un à l'autre le cathéter est dans un état d'administration de la substance.

9. Système de perfusion selon la revendication 1,
**caractérisé en ce que** le cathéter interne comprend deux lumières.

10. Système de perfusion selon la revendication 1,
**caractérisé en ce que** toutes les surfaces en contact avec des substances actives dans le dispositif de cathéter sont faites ou couvertes de tétrafluoropolyéthylène (Teflon®).

11. Système de perfusion selon la revendication 1,
**caractérisé en ce que** le volume d'une impulsion du liquide de la substance est approximativement le même que le volume défini dans la dite lumière du cathéter externe entre l'ouverture d'écoulement du cathéter interne et l'ouverture d'écoulement du cathéter externe.
